# EUROPEAN PATENT APPLICATION

(11) **EP 1 738 757 A1**
(43) Date of publication of application: **03.01.2007**
(21) Application number: 05013790.0
(22) Date of filing: 27.06.2005
(51) Int. Cl.: A61K 31/216, A61K 31/22, A61K 31/40, A61K 31/366, A61K 31/404, A61K 31/4402, A61K 31/785, A61P 9/10

(54) **Prevention of secondary stroke with cholesterol lowering agents**

(71) Applicant: Solvay Pharmaceuticals GmbH, 30173 Hannover (DE)
(72) Inventor: The designation of the inventor has not yet been filed
(74) Representative: Gosmann, Martin

(57) **Abstract**

The present invention relates to the use of cholesterol lowering agents for prevention of secondary strokes, in particular the use of statins and fibrates: including lovastatin, fluvastatin, pravastatin, atorvastatin, fenofibrate, gemfibrozil, etofibrate and colestyramine.

## Description

The present invention relates to the use of cholesterol lowering agents for prevention of subsequent strokes.

Each year approximately five million people worldwide die of a stroke. Each year approximately fifteen million people have a non-fatal stroke, approximately one third of them sustaining substantial permanent health damage. Strokes are thus the third leading cause of death but the main cause of permanent health damage in adults.
Of the survivors of an acute (= first) stroke, approximately 20% have a subsequent or secondary stroke within the next five years. A stroke in the sense of the present invention is understood to refer to a cerebrovascular event, such as a transient ischemic attack, cerebral ischemia and intracerebral hemorrhaging.

It is known that high blood pressure is one of the most important risk factors for an acute (first) stroke. Consequently, the risk of hypertensive patients suffering an acute stroke can be reduced statistically significantly through antihypertensive therapy. Antihypertensive therapy for the purpose of preventing acute strokes can be administered with comparable results by using compounds such as those from the pharmacological classes of beta-blockers, calcium antagonists, diuretics or in some cases even a combination therapy, including the use of compounds from the ACE inhibitor class. The suitability of the AT₁-receptor antagonist candesartan for antihypertensive therapy during the acute phase of a stroke has been the subject of a clinical trial (ACCESS Study, see J. Schrader et al., Basic Res. Cardiol. 93, Suppl. 2 (1998), 69-78), for example.

However, in prevention of sübseqüent (e.g., secondary) strokes, no clear correiation can be found between lowering elevated blood pressure and reducing a (male or female) patient's risk of suffering a subsequent stroke. A subsequent stroke is a stroke which occurs in the same person after at least one prior initial stroke. In particular, a secondary stroke should be understood to refer to a new stroke occurring after just one prior stroke in the same patient.

To investigate the circumstances that can influence the risk of a subsequent stroke, a long-term clinical study has been conducted on stroke patients. The hypertensive and nonhypertensive patients participating in this study received as the active ingredient the ACE inhibitor perindopril either alone (= ACE monotherapy) or in combination with the diuretic indapamide (=ACE combination therapy) (= PROGRESS Study, see, for example, The PROGRESS Collaboration Group, The Lancet 358 (2001), 1033-1041). In this clinical study, it was found that the blood pressure of patients in the hypertensive group could be lowered to a comparable extent by ACE monotherapy as well as ACE combination therapy. Nevertheless, the risk of suffering a subsequent stroke dropped significantly only in the subgroup of hypertensive patients treated with the ACE combination therapy, whereas for the subgroup of hypertensive patients treated with ACE monotherapy, the risk of suffering a subsequent stroke did not differ from the corresponding risk in the placebo patient group. Another finding of the PROGRESS study was that the reduction in risk, if any, of suffering a subsequent stroke was of approximately the same order of magnitude in both the hypertensive and nonhypertensive patient groups.

Furthermore, in the state of the art it is reported about improved posthypoxic recovery in vitro on treatment with drugs used for secondary stroke prevention (Huber Roman; Riepe Matthias, Neuropharmacology, (2005 Mar) 48 (4) 558-65). It was found that besides aspirin several new drugs for inhibition of platelet aggregation and 3-hydroxy-3-methylglutaryl-coenzyme A (HMG-CoA) reductase inhibition are used in secondary stroke prevention. Pharmacology and clinical effects, however, are not fully explained by current understanding of underlying mechanisms The authors present data that demonstrate a shared neuroprotective effect of agents known to inhibit platelets (acetylsalicylic acid, clopidogrel, and ticlopidine) and HMG-CoA reductase (atorvastatine). The time course of this neuroprotective action in the current experimental study (onset within an hour, duration of several hours in contrast to several days) resembles clinical practice in dosing these substances. It is hypothesized that an increase of hypoxic tolerance resulting from mild mitochondrial inhibition by these substances is a principal constituent of the effectiveness of these drugs.

A coordinated stroke treatment program to prevent recurrent thromboembolic events is described by Ovbiagele B; Saver J L; Fredieu A; Suzuki S; McNair N; Dandekar A; Razinia T; Kidwell C S, in Neurology, (2004 Oct 12) 63 (7) 1217-22. The objective of the investigations was to assess the impact of the Preventing Recurrence of Thromboembolic Events through Coordinated Treatment (PROTECT) Program on achievement of its eight secondary prevention goals at the time of discharge. Achievement rates for the eight program goals at time of discharge were compared in all patients discharged from a university hospital-based stroke service with a diagnosis of ischemic stroke or TIA during a 1-year period after implementation of the PROTECT Program vs rates obtained from a comparable group of patients admitted to the same service during the preceding year.

RESULTS: Demographic and medical features were comparable in the baseline and intervention cohorts for all patients with cerebral ischemia presumed due to large-vessel atherosclerosis or small-vessel disease (baseline year n = 117, intervention n = 130). Implementation rates in patients without specific contraindications increased for all four medication goals: 97 to 100% for antithrombotic agents, 68 to 97% for statins, 42 to 90% for angiotensin-converting enzyme inhibitors/angiotensin receptor blockers, and 14 to 70% for diuretics. Although data were not collected on baseline lifestyle instruction rates, instruction in the program's four lifestyle interventions was achieved by discharge in 100% of the intervention cohort. It was concluded that the implementation of this single-center, systems-based, in-hospital program to initiate secondary stroke prevention therapies was associated with a substantial increase in treatment utilization at the time of hospital discharge.

The use of statins for secondary stroke prevention in patients without known coronary heart disease: the jury is still out, in the state of the art, as reported by Goldstein Larry B; Amarenco Pierre; Bogousslavsky Julien; Callahan Alfred S; Hennerici Michael G; Welch K Michael A; Zivin Justin; Sillesen Henrik in Cerebrovascular Diseases (Basel, Switzerland), (2004) 18 (1) 1-2; Electronic Publication: 2004-05-19; Journal code: 9100851. ISSN: 1015-9770.

The medical prevention of secondary stroke as a cardiologist's perspective was discussed by Ezekowitz Michael in Clinical Cardiology, (2004 May) 27 (5 Suppl 2) II36-42. Ref: 47. Post-stroke patients are at greatest risk from a second stroke rather than an event in another vascular bed. Thus treatment strategies must be aimed at preventing recurrent stroke. Recent evidence suggests that the pathophysiology underlying stroke may differ from that of coronary artery disease. Secondary events may replicate primary events, and treatment strategies for patients who experienced an initial stroke must therefore focus on preventing recurrent stroke. Medical strategies for secondary stroke prevention focus on four areas: control of hypertension, control of blood lipids, anticoagulant therapy, and treatment with antiplatelet agents such as aspirin, clopidogrel, and aspirin combined with extended-release dipyridamole, all of which the American College of Chest Physicians deems acceptable for first-line treatment for preventing secondary stroke.

Secondary stroke prevention was also describeb in a review of clinical trials (Elkind Mitchell S V, Clinical cardiology, (2004 May) 27 (5 Suppl 2) 1125-35. Ref: 71). Patients who experience a stroke or transient ischemic attack (TIA) are at high risk for subsequent vascular events, most commonly stroke. This article focuses on clinical trials examining secondary prevention of stroke and reviews the various commonly used methods of stroke prevention: surgical approaches, antihypertensive treatment, lipid- and cholesterol-lowering medications, anticoagulant therapies, and antiplatelet therapies.

A further article deals with the tracking trends in secondary stroke prevention strategies (Carswell Jody L; Beard Kemberley A; Chevrette Michele M; Pardue Carol N; Hess David C; Fagan Susan C, Annals of pharmacotherapy, (2004 Feb) 38 (2) 215-9; Electronic Publication: 2003-12-23). Stroke is here defined as a rapid onset of isolated neurologic dysfunction and is the leading cause of disability in adults, as well as the third-leading cause of death in the US. Nearly 600,000 cases of stroke are reported annually. OBJECTIVE: To quantify the impact of emerging evidence on the use of secondary stroke prevention strategies in patients discharged from a specialized stroke center in 2002 compared with those discharged in 2000. METHODS: Using a retrospective approach, data were collected on the first 100 patients cared for by the stroke service from July 1, 2000, to December 31, 2000. Using a prospective approach, the goal was to enroll 100 patients with a diagnosis of ischemic stroke and telephone the patients 1 and 3 months after discharge to determine patient adherence and persistence rate of medications. This is an interim report of the first 20 patients enrolled. Both studies resulted in populations of 55% women and 45% men. The most common risk factor was hypertension, with 59% on admission in 2000 and 75% in 2002. Use of antithrombotics (98% in 2000 to 100% in 2002) and antihypertensives (67% in 2000 to 90% in 2002) was high in both studies and not significantly different.

The 2 most significant changes in prescribing were increased use of statins (13% in 2000 to 50% in 2002, p = 0.0007) and combination clopidogrel and aspirin use (0% in 2000 to 20% in 2002, p = 0.001). At 3 months, 66.7% of patients were on the same antithrombotic medication as at discharge. CONCLUSIONS: The dedicated stroke service was able to effectively translate emerging evidence and guidelines into practice and significantly change the use of prescribed medications for secondary stroke prevention.

Furthermore, the design and baseline characteristics of the stroke prevention were described by aggressive reduction in cholesterol levels (SPARCL) study.
(Erratum in: Cerebrovasc Dis. 2004;17(1):91-2; Amarenco Peirre; Bogousslavsky Julien; Callahan Alfred S; Goldstein Larry; Hennerici Michael; Sillsen Henrik; Welch Michael A; Zivin Justin; Cerebrovascular Diseases (Basel, Switzerland), (2003) 16 (4) 389-95). Evidence suggests that statin therapy reduces the risk of stroke in patients with coronary heart disease (CHD), but its benefit for patients with cerebrovascular disease and no history of CHD remains uncertain. The Stroke Prevention by Aggressive Reduction in Cholesterol Levels (SPARCL) Study is a prospective, multi-centre, double-blind, randomised, placebo-controlled trial designed to evaluate the effects of atorvastatin 80 mg/day in patients who previously experienced a stroke or transient ischaemic attack, but who have no known CHD. A total of 4732 patients have been enrolled, and the data collection phase of the study is expected to be completed by October 2004. SPARCL is the first study primarily designed to prospectively evaluate the effect of statin treatment in secondary stroke prevention.

It has now surprisingly been found that by administering cholesterol lowering agents, preferably statins or fibrates, as the only active ingredient (stain monotherapy), a patient's risk of suffering a subsequent stroke can be reduced significantly. Therefore, the present invention relates to the use of cholesterol lowering agents, preferably statins, for prevention of subsequent strokes.

The cholesterol lowering agents, in particular the statins or fibrates, are well known in the state of the art regarding their pharmacological activity; however it was unkown heretofore to use cholesterol lowering agents, in particular the statins or fibrates, in large mammals, humans in particular, as preferred and sole agents in the prevention of subsequent or secondary stroke. Particularly suitable cholesterol lowering agents, in particular the statins or fibrates, for use in the present invention include lipid lowering agents of the ATC-C-1 0 Group, in particular statins like lovastatin, fluvastatin, pravastatin, atorvastatin; and fibrates like fenofibrate, gemfibrocil, etofibrate; and colestyramin. These agents and their physiologically acceptable free acids, if applicable, or acid addition salts may be used according to the invention. It should be understood, however, that any other cholesterol lowering agents, in particular any other statins or fibrate, also may be used within the scope of the present invention.

Prevention is understood to refer to preventive care for patients to prevent or at least inhibit subsequent strokes, in particular preventive care to inhibit a subsequent stroke after just one prior acute (first) cerebrovascular event. Prevention of a subsequent stroke after just one prior initial stroke is usually referred to as secondary stroke prevention. Use of in particular the statins or fibrates, according to this invention for secondary stroke prevention is preferred. Usually a patient requiring subsequent stroke prevention will take an amount of at least one cholesterol lowering agent, in particular a statin or fibrate, adequate to reduce his risk of suffering a subsequent stroke and will take it for a lengthy period of time, under some circumstances permanently, as maintenance therapy.

A comparative analysis of anonymisized data collected from a database reporting about the treatments of patients who have suffered stroke is being conducted, investigating the suitability of a variety of agents from various pharmacological classes to reduce a patient's risk of suffering a subsequent stroke. The primary target criterion of the analysis is the evaluation of the hazard ratio and the total mortality in view of the total number of cardiovascular and cerebrovascular events (strokes) in the sense of this invention. The hazard ratio shows the change in risk for the respective medication, e.g. if a drug is administered. The variety of drug included heart therapy, antihypertensives, diuretics, peripheral vasodilators vasoprotectives, other cardiovascular agents, beta blockers, calcium antagiónists, rennin angiotensin medication and cholesterol lowering agents. In this analysis it was found that among the agents used in the treatments to prevent secondary stroke the class of cholesterol lowering agents, in particular of statin or fibrate, was surprisingly and particularly outperforming in reducing the patient's risk of suffering a subsequent stroke after the initial stroke. The hazard ratio was decreased to 87.4 % with a significance of p < 0.001. In case of cholesterol lowering agents, in particular a statin or fibrate, the hazard ratio was 0.874 compared to a range of 0.966 to 1.142 for the other medications indicated above. Furthermore, it was found that patient's mortality risk is significantly decreased to 67.7% (p < 0.001) if a drug of the class of cholesterol lowering agents, in particular a statin or fibrate, was administered to the patient.

Selection criteria were one or several strokes excluding a time window of 30 days after the first stroke. This implies that the patient must have survived the initial stroke by at least 30 days, and that events in a 30 days time window after an initial stroke are not regarded as secondary stroke. The selection event is the second stroke with re-admittance to the hospital because of diagnosis of stroke. The data of 42,628 patients (42 % male / 58 % female) were analyzed, involving 72,000 stroke events and 4 million drug prescriptions. According to the above inclusion criteria 38.605 patients were selected from which 6.476 patients suffered from subsequent or secondary stroke. The remaining patients were not sent to the hospital for a second time or already in a time period of less than 30 days after initial stroke. The results and findings according to the present invention are furthermore depicted in the Figures below

It can be seen from the analysis findings presented above that a significant reduction in a patient's risk of having a subsequent stroke can be achieved through cholesterol lowering agents monotherapy, in particular monotherapy with a statin or fibrate. This success achieved with cholesterol lowering agents monotherapy, in particular monotherapy with a statin or fibrate, in the inhibition of subsequent strokes and reduction of mortality is much better than the success achieved with methods investigated so far.

It can thus be concluded that cholesterol lowering agents, in particular statins or fibrates, yield an especially pronounced reduction in a patient's risk of suffering a subsequent stroke or mortality rik, and that cholesterol lowering agents, in particular statins or fibrates, are especially effective in preventing or inhibiting subsequent strokes, in particular for secondary stroke inhibition, and reducing mortality risk involved in stroke events. Instead of prescribing cholesterol lowering agents, in particular statins or fibrates, only after a subsequent or secondary stroke, according to the invention, therefore, it is beneficial to administer cholesterol lowering agents, in particular statins or fibrates, already after the initial stroke in order to prevent the risk of subsequent or secondary stroke and the mortality risk caused by subsequent or secondary stroke.

The doses of the cholesterol lowering agents, in particular of the statins or fibrates, used may vary individually and will of course also vary according to the type of substance used and the form of administration. In general, however, the pharmaceutical dosage forms known from cholesterol lowering therapy, in particular cholesterol therapy with a statin or fibrate, and having an usual active ingredient content per single dose found to be suitable for administration of said agents to large mammals, in particular humans.

As medicinal agents, the cholesterol lowering agents, in particular the statins or fibrates, can be formulated with conventional pharmaceutical excipients in galenical preparations such as tablets, capsules, suppositories or solutions. These galenical preparations can be produced according to known methods using conventional solid or liquid vehicles such as lactose, starch or talc or liquid paraffins and/or using conventional pharmaceutical excipients, e.g., tablet disintegrants, solubilizers or preservatives. The production of galenical preparations of cholesterol lowering agents, in particular of the statins or fibrates, is known per se, e.g., from the public sources of the production processes known to the skilled artisan.

The pharmaceutical preparations according to this invention can be produced by methods known tom the skilled artisan or similar to those already known.

The following example is presented to describe the present invention in greater detail without restricting its scope in any way.

### Description of Figures:

### Fig. 1:

Time window until subsequent (secondary) stroke (in years); cumulative survival rate depending on gender. Patients were in average 73 years old at time of initial stroke (average fro male 69 years, for female 76 years).

### Fig. 2:

Reduction of patient's risk of subsequent (secondary) stroke with cholesterol lowering agent.

### Fig. 3:

Reduction of patient's mortality risk in subsequent (secondary) stroke with cholesterol lowering agent.

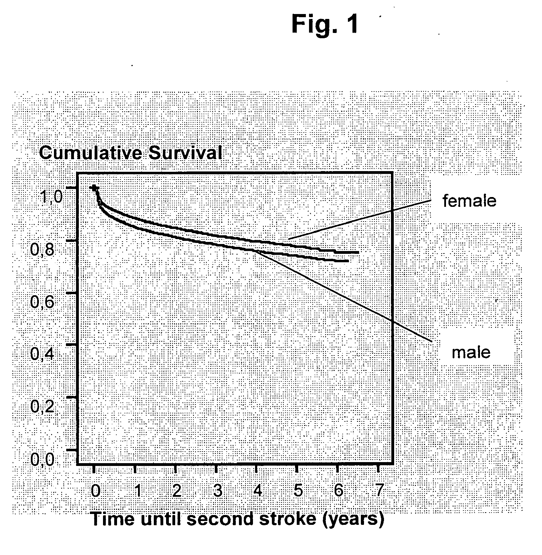

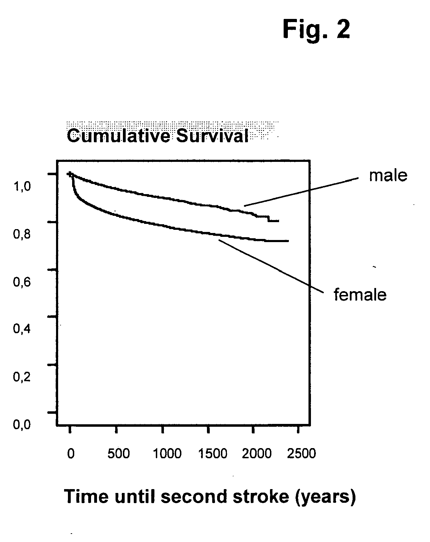

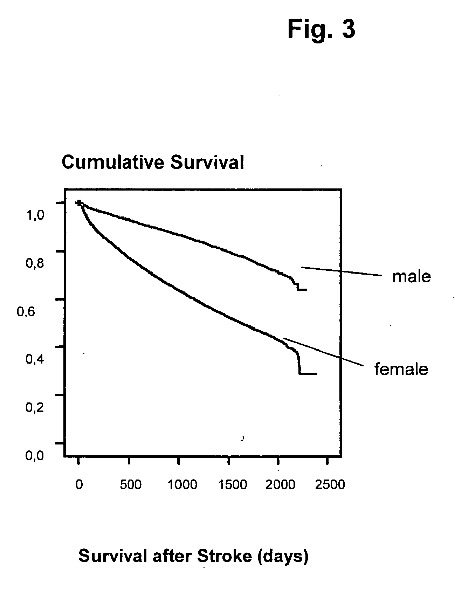

## Claims

1. Use of cholesterol lowering agents, in particular the statins or fibrates, a free acid thereof or a physiologically acceptable acid addition salt thereof in the manufacture of a medicament for the prevention of subsequent stroke attacks.

2. Use of cholesterol lowering agents, in particular the statins or fibrates, according to claim 1, for prevention a secondary stroke attack.

3. Use according to claim 1 or 2, wherein the cholesterol lowering agent is selected from the group of statins, fibrates, and colestyramin.

4. Use according to claim 3, wherein the cholesterol lowering agent is selected from the group of statins or a physiologically acceptable acid addition salt thereof.

5. Use according to claim 4, wherein the statin is lovastatin, fluvastatin, pravastatin, atorvastatin; or a physiologically acceptable acid addition salt thereof.

6. Use according to claim 4, wherein the fibrate is like fenofibrate, gemfibrocil, etofibrate; a free acid thereof or a physiologically acceptable acid addition salt thereof.

7. Use of AT₁-receptor antagonist for prevention of secondary stroke attacks.
